(19)

**Europäisches Patentamt**
**European Patent Office**
**Office européen des brevets**

(11) **EP 3 643 333 B1**

(12) **EUROPEAN PATENT SPECIFICATION**

(45) Date of publication and mention
of the grant of the patent:
**10.05.2023  Bulletin 2023/19**

(21) Application number: **17914494.4**

(22) Date of filing: **27.07.2017**

(51) International Patent Classification (IPC):
*A61L 27/36* (2006.01)     *A61L 27/42* (2006.01)
*A61L 27/24* (2006.01)     *A61L 27/54* (2006.01)
*A61C 8/00* (2006.01)     *A61C 13/00* (2006.01)
*A61L 27/48* (2006.01)

(52) Cooperative Patent Classification (CPC):
(C-Sets available)
**A61C 8/0006; A61L 27/36; A61L 27/42;**
**A61L 27/48; A61L 27/54**     (Cont.)

(86) International application number:
**PCT/KR2017/008112**

(87) International publication number:
**WO 2018/235984 (27.12.2018 Gazette 2018/52)**

(54) **INTEGRAL BIOMATERIAL FOR REGENERATION OF BONE TISSUE AND FABRICATION METHOD THEREFOR**

INTEGRALES BIOMATERIAL ZUR REGENERATION VON KNOCHENGEWEBE UND HERSTELLUNGSVERFAHREN DAFÜR

BIOMATÉRIAU INTÉGRÉ POUR LA RÉGÉNÉRATION DE TISSU OSSEUX ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2017  KR 20170077400**

(43) Date of publication of application:
**29.04.2020  Bulletin 2020/18**

(73) Proprietors:
• **Nibec Co., Ltd.**
**Chungcheongbuk-do 27816 (KR)**
• **Seoul National University R&DB Foundation**
**Seoul 08826 (KR)**

(72) Inventors:
• **PARK, Yoon Jeong**
**Seoul 08291 (KR)**
• **CHUNG, Chong-Pyoung**
**Seoul 05618 (KR)**
• **LEE, Jue-Yeon**
**Gwacheon-si**
**Gyeonggi-do 13831 (KR)**

• **CHAE, Ju Yeon**
**Seoul 07295 (KR)**

(74) Representative: **Mewburn Ellis LLP**
**Aurora Building**
**Counterslip**
**Bristol BS1 6BX (GB)**

(56) References cited:
**WO-A1-99/19005          WO-A1-2018/209101**
**KR-A- 20140 025 221     KR-A- 20160 080 512**
**KR-A- 20160 148 551     US-A1- 2015 257 870**
**US-A1- 2016 106 885     US-A1- 2016 106 885**

• **DATABASE WPI Week 201046 Thomson Scientific, London, GB; AN 2010-G88800 XP002801844, & JP 2010 131130 A (UNIV TOKYO) 17 June 2010 (2010-06-17)**

Remarks:
The complete document including Reference Table(s) and the Sequence Listing(s) can be downloaded from the EPO website

EP 3 643 333 B1

(52) Cooperative Patent Classification (CPC): (Cont.)

C-Sets
**A61L 27/48, C08L 89/06**

C-Sets
**A61L 27/48, C08L 89/06**

**Description**

[Technical Field]

The present invention relates to an integrated biomaterial for bone tissue regeneration and a method of preparing the same, and more particularly to an integrated biomaterial for bone tissue regeneration including a lower structure consisting of an extracellular matrix protein and a bone mineral and an upper layer consisting of an extracellular matrix protein, and a method of preparing the same.

[Background Art]

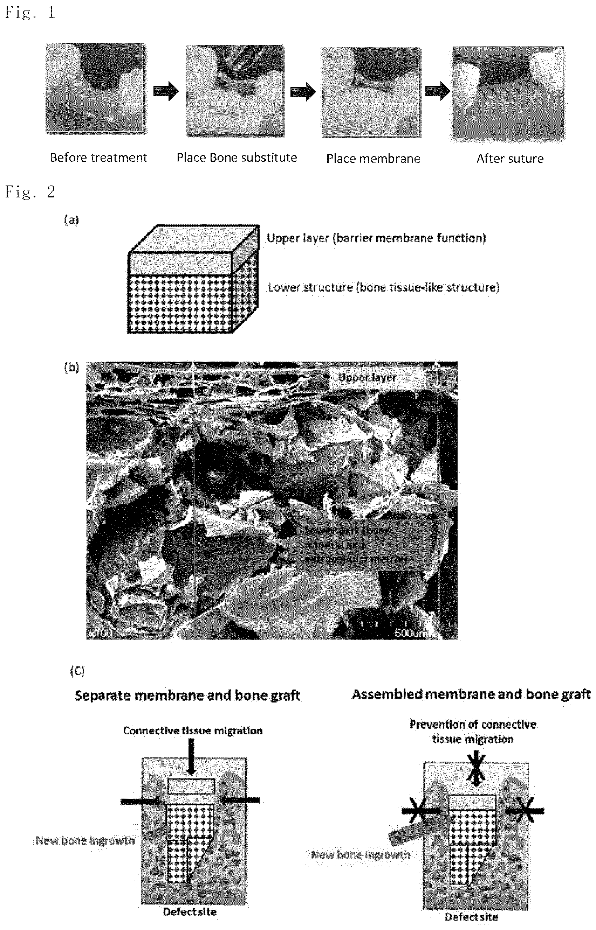

The periodontal tissue supporting the teeth broadly consists of alveolar bone, connective tissue constituting the periodontal membrane between the alveolar bone and the tooth, epithelial tissue and periodontal ligament tissue . The loss of alveolar bone due to the progression of periodontitis is accompanied by loss of periodontal ligament tissues, and normal recovery of alveolar bone and periodontal ligament tissues is impossible due to overgrowth of connective tissues at the site of tissue loss after periodontal treatment. In addition, even when new bone is generated, the periodontal ligament tissue may not be normally differentiated, resulting in loss of dental function. Therefore, to address these problems, an artificial barrier membrane is used together with bone substitute grafting as an alveolar bone regeneration surgery. When a bone graft material is used alone, there is a drawback in that the bone graft material is difficult to maintain at a graft site, and thus tissue regeneration is induced using a method of filling a bone loss site with a bone graft material and placing a shielding (barrier) membrane thereon and suturing the membrane (see FIG. 1). When a bone graft material and a barrier membrane are used at the same time, the bone graft material implanted on a lower side and the barrier membrane on an upper side must be in close contact with each other to facilitate bone regeneration, thus increasing the success rate of bone grafting. In contrast, if there is a gap between the bone graft material and the barrier membrane, the success rate of bone regeneration is lowered. However, applying separate products of a bone graft material and a barrier membrane makes it impossible to achieve perfect close contact between the two products. Therefore, if an integrated product having both a bone graft material and a barrier membrane function is developed, the success rate of bone regeneration can be increased. It may be possible to simultaneously obtain osteoconductivity of the bone graft material and a connective tissue preventive effect of the barrier membrane in one product, and thus convenience of use may be enhanced (see FIGS. 2A and 2B).

Until recently, the most frequently used graft material for bone regeneration surgery includes an autogenous bone graft material, an allogenic bone graft material, a xenogenic bone graft material, and a synthetic bone graft material, but autogenous bone requires secondary surgery for bone collection so that it is difficult to obtain such bone, allogenic bone is problematic in the possibility of contamination with a disease, and synthetic bone has low biocompatibility with natural bone tissue, and thus xenogenic bone is widely used.

In the case of barrier membranes, it has been reported that barrier membranes have been used to induce regeneration of periodontal tissue effectively over the past 20 years (J. Gottlow, et al., J. Clin. Perio, 13, (1986) pp. 604~616), and since then, research on tissue induction and regeneration has been carried out using various materials as barrier membranes. Recently, among biodegradable barrier membranes, barrier membranes made of collagen are the most widely used, but have limitations such as weak mechanical strength and incomplete close contact with a bone graft material.

Meanwhile, bones and teeth in the human body contain approximately 80% minerals and water and 20% organic matter, and 80% of organic matter consists of collagenous proteins and 20% thereof consists of non-collagenous proteins. These protein components not only contribute to maintaining the production of hard tissue and structural strength and elasticity, but also act as a matrix for inducing the adhesion of hard tissue-forming cells such as osteoblasts and for orienting inorganic ion components constituting hard tissue components (Anselme, Osteoblast adhesion on biomaterials, Biomaterials, 21 (7): 667-81, 2000).

Existing xenogenic bone-derived bone graft materials contain only mineral components, from which proteins are completely removed, and thus do not have the same structure as that of natural bone. To address this problem, a biomaterial to which proteins constituting bone tissue, such as collagen, are introduced is being developed. However, it is impossible to prepare a stable biomaterial by simply mixing collagen and a bone graft material, and existing biomaterial preparation methods have limitations in preparation of a biomaterial having a barrier membrane function.

Meanwhile, a method of separately preparing an upper layer and a lower structure and binding the upper layer and the lower structure via covalent bonding using a chemical crosslinking agent in the final product stage has been attempted, but the crosslinking reaction hardly occurs in a solid phase state so that sufficient bonding cannot be formed, and when a product is allowed to sufficiently absorb a physiological saline solution or be hydrated therewith for use, there is a problem such as separation between the upper layer and the lower structure. In addition, when a chemical crosslinking agent is used, there should be no chemical crosslinking agent remaining in the product, but if it remains, toxicity may be caused at the graft site, and thus the chemical crosslinking agent exhibits an adverse effect in terms of safety. In addition, when the lower structure and the upper layer are separately implanted, a space is generated between the two structures, and thus connective tissue may infiltrate not only from the upper side but also from the side surface thereinto, thereby interfering with a bone regeneration process proceeding from the lower side, resulting in significantly reduced bone regeneration efficiency. Document US 2016/106885 A1 discloses an integrated biomaterial for bone tissue regeneration, the integrated biomaterial comprising: a lower structure comprising an extracellular matrix protein and a bone mineral component; and an upper layer comprising collagen. There is no indication of specific amount of collagen and ratio between the lower and upper part of the implant.

Therefore, to address the above-described problems, there is a need to develop an integrated biomaterial which not only realizes a bone tissue environment, but also has a barrier membrane function capable of preventing infiltration of connective tissue.

Therefore, as a result of having made intensive efforts to address the above-described conventional problems, the inventors of the present invention developed an integrated biomaterial to which extracellular matrix and bone mineral components are organically bound so as to have a composition similar to that of bone tissues, and having a barrier membrane function, and confirmed that such an integrated biomaterial has an excellent effect on bone regeneration, thus completing the present invention.

[Disclosure]

[Technical Problem]

Therefore, the present invention has been made in view of the above problems, and it is an object of the present invention to provide an integrated biomaterial for bone tissue regeneration which not only realizes a bone tissue environment, but also prevents infiltration of connective tissues to thereby exhibit maximized bone tissue regeneration capability, and a method of preparing the same.

[Technical Solution]

In accordance with the present invention, the above and other objects can be accomplished by the provision of an integrated biomaterial for bone tissue regeneration, the integrated biomaterial comprising: a lower structure including collagen and a bone mineral component; and an upper layer including collagen, wherein an amount ratio (weight ratio) of the collagen of the upper layer to the collagen of the lower structure ranges from 0.13-1.3:1.

In accordance with an aspect of the present invention, the above and other objects can be accomplished by the provision of a use of an integrated biomaterial for bone tissue regeneration, the integrated biomaterial comprising: a lower structure including collagen and a bone mineral component; and an upper layer including collagen, wherein an amount ratio (weight ratio) of the collagen of the upper layer to the collagen of the lower structure ranges from 0.13-1.3:1.

Disclosed, but not part of the present invention, there is provided a bone tissue regeneration method including implanting, into an individual in need of bone tissue regeneration, an integrated biomaterial comprising: a lower structure including an extracellular matrix protein and a bone mineral component; and an upper layer including an extracellular matrix protein.

In accordance with a further aspect of the present invention, there is provided a method of preparing an integrated biomaterial for bone tissue regeneration comprising: a lower structure including collagen and a bone mineral component; and an upper layer including collagen, the method comprising: (a) molding a lower structure mixture including collagen and bone mineral particles; (b) aligning a structure of the lower structure mixture including collagen and bone mineral particles; (c) placing an upper layer including collagen thereon; (d) binding the upper layer and the lower structure; (e) lyophilizing the resulting structure; and (f) thermally cross-linking the collagen of the upper layer, wherein an amount ratio (weight ratio) of the collagen of the upper layer to the collagen of the lower structure ranges from 0.13-1.3:1.

[Description of Drawings]

The above and other objects, features and other advantages of the present invention will be more clearly understood from the following detailed description taken in conjunction with the accompanying drawings, in which:

FIG. 1 is a schematic view illustrating a general bone graft procedure, wherein, after being first filled with a bone graft material, a barrier membrane is placed thereon and sutured;

FIG. 2A is a schematic view of an integrated biomaterial in which a bone graft material and a barrier membrane function are integrated, FIG. 2B is a differential scanning electron microscope image of an integrated biomaterial in which a bone graft material and a barrier membrane function are integrated, and FIG. 2C illustrates a process of bone regeneration using graft material and a barrier membrane separately or an integrated biomaterial in which a bone graft material and a barrier membrane function are integrated;

FIG. 3 illustrates differential scanning microscope images showing integrated biomaterials prepared according to Examples 1, 2, and 3, wherein arrows indicate a collagen layer;

FIG. 4 illustrates the results of observing the degree of degradation of an upper collagen layer of each of the integrated biomaterials of Examples 1, 2, and 3 by collagenase, wherein arrows indicate a collagen layer; and

FIG. 5 illustrates the results of observing the degree of bone regeneration of each of the integrated biomaterials of Examples 1, 2, and 3 after being implanted into bone defect sites of rabbits, wherein arrows indicate a collagen layer, G denotes a bone graft material, and NB denotes new bone.

[Detailed Description and Exemplary Embodiments]

Unless defined otherwise, all technical and scientific terms used herein have the same meaning as is commonly understood by one of ordinary skill in the art to which the present invention pertains. Generally, the nomenclature used herein is well known in the art and commonly used.

In the present invention, it was confirmed that, when an integrated biomaterial prepared by forming an upper layer formed of an extracellular matrix protein on a lower structure consisting of an extracellular matrix protein and a bone mineral is used as a bone graft material, the lower structure realizes a bone tissue environment to facilitate the regeneration of new bone, and the upper layer enables the bone graft material to be stably maintained on a bone defect site and realizes a natural bone tissue environment at a graft site by preventing the infiltration of epithelial tissue or connective tissue, thereby maximizing bone tissue regeneration capacity.

In addition, the integrated biomaterial may be prepared by inducing physical binding between the two structures in an initial preparation process without using an additional reagent such as a chemical crosslinking agent, other than main raw materials, and thus toxicity problems caused by a chemical crosslinking agent may be prevented, and an upper layer and a lower layer are not separated from each other even after hydration to thus also achieve structural stability.

In addition, compared with a case in which, when a lower structure and an upper layer are separately implanted, connective tissue infiltrates into a space between the two structures and thus interferes with a bone regeneration process, the integrated biomaterial has no space between the two structures, and thus connective tissue does not infiltrate into a side surface thereof so that the bone regeneration process proceeding from the bottom thereof is smoothly and effectively induced (see FIG. 2C).

Therefore, an embodiment of the present invention relates to an integrated biomaterial for bone tissue regeneration comprising a lower structure including collagen and a bone mineral component and an upper layer including collagen, wherein an amount ratio (weight ratio) of the collagen of the upper layer to the collagen of the lower structure ranges from 0.13-1.3:1.

Disclosed but not part of the present invention relates to a use of an integrated biomaterial for bone tissue regeneration, the integrated biomaterial comprising: a lower structure including an extracellular matrix protein and a bone mineral component; and an upper layer including an extracellular matrix protein.

Disclosed but not part of the present invention relates to a method of regenerating bone tissue, comprising implanting, into an individual in need of bone tissue regeneration, an integrated biomaterial comprising: a lower structure including an extracellular matrix protein and a bone mineral component; and an upper layer including an extracellular matrix protein.

Another embodiment of the present invention relates to a method of preparing the integrated biomaterial for bone tissue regeneration, comprising: (a) molding a lower structure mixture including collagen and bone mineral particles; (b) aligning a structure of the lower structure mixture including collagen and bone mineral particles; (c) placing an upper layer including an extracellular matrix protein thereon; (d) binding the upper layer and the lower structure; (e) lyophilizing the resulting structure; and (f) thermally cross-linking the collagen of the upper layer, wherein an amount ratio (weight ratio) of the collagen of the upper layer to the collagen of the lower structure ranges from 0.13-1.3:1.

In the present invention, the upper layer including collagen must be organically bound to the lower structure including collagen and a bone mineral component, which is a bone tissue-like biomaterial, and must not be separated therefrom when applied *in vivo*. In addition, since the upper layer including collagen must be maintained for at least one week when implanted *in vivo,* the degree of degradation by a protease such as collagenase should be 10% (w/w) or less with respect to the total weight. In the present invention, in all of the integrated biomaterials prepared according to Examples 1 to 3, the collagen degradation rate of the upper layer by collagenase was in the range of 2.41% (w/w) to 5.90% (w/w) at the point of two weeks after collagen degradation, from which it was confirmed that the retention of the upper layer as a barrier membrane could last one week or longer.

In process (a) of the present invention, a mold is filled with a lower structure mixture including collagen and bone mineral particles and molded.

In process (b) of the present invention, the alignment of the structure of the lower structure mixture of collagen and bone mineral particles means that hydrophobic bonds, hydrogen bonds, or the like are formed between protein chains as a distance between the protein chains becomes narrow, thereby aligning protein chain arrangement, resulting in structural stabilization.

The concentration of collagen used in process (c) of the present invention ranges from 0.5% (w/w) to 10% (w/w), more preferably in the range of 2% (w/w) to 5% (w/w), with respect to a total concentration of the biomaterial.

Process (d) of the present invention is a process of binding the upper layer (i.e., collagen) and the lower structure (i.e., a mixture including collagen and bone mineral particles) through gelation using a strong base, and the strong base may be selected from the group consisting of sodium hydroxide, potassium hydroxide, calcium hydroxide, barium hydroxide, ammonium hydroxide, calcium carbonate, potassium carbonate, and ammonia, but the present invention is not limited thereto.

Process (e) of the present invention may be performed by freezing at -1.5 °C for 2 hours or longer, followed by freezing at -20 °C at a freezing rate of 1 °C/min, but a lyophilization method commonly used in the art may be applied.

Process (f) of the present invention is intended to extend the degradation time by dehydrothermal treatment of the collagen of the upper layer, and thermal crosslinking may be performed by treatment thereof at 140 °C to 160 °C for 48 hours to 168 hours.

Meanwhile, the method of preparing the integrated biomaterial for bone tissue regeneration may further include, after process (e), (g) adding an antimicrobial or anti-inflammatory functional material; and (h) lyophilization.

In the present invention, the extracellular matrix protein of the lower structure and the upper layer is collagen. As such collagen, one derived from a human or an animal may be used. In the case of collagen, it is preferable to use type 1 or type 3 isolated from pig skin.

In the present invention, the bone mineral component may be one or more selected from the group consisting of living organism-derived bone mineral powder which originate from allogenic bone or xenogenic bone, synthetic hydroxyapatite, and tricalcium phosphate micropowder.

In the present invention, a ratio of the bone mineral component to the extracellular matrix protein may be varied, and the content of the bone mineral component is preferably 80 wt% or more, more preferably in the range of 80 wt% to 95 wt%, with respect to the total weight of the integrated biomaterial. A total content of the extracellular matrix protein of the lower structure and the extracellular matrix protein of the upper layer is preferably 5 wt% or more, more preferably in the range of 5 wt% to 20 wt%, with respect to the total weight of the integrated biomaterial.

Meanwhile, an amount ratio (weight ratio) of the extracellular matrix protein of the upper layer to the extracellular matrix protein of the lower structure ranges from 0.13-1.3: 1, and particularly, when the content ratio (weight ratio) of the extracellular matrix protein of the upper layer to the extracellular matrix protein of the lower structure is 0.5:1, it is the most preferable in terms of a significant increase in new bone formability. In this case, when the content of the collagen of the upper layer is less than 0.13 parts by weight with respect to 1 part by weight of the collagen of the lower structure, the upper layer is too thin (about 200 $\mu$m or less), and thus is unable to function as a barrier membrane for preventing infiltration of connective tissue, and accordingly, this case is not suitable for use as an integrated biomaterial for bone tissue regeneration. When the content of the collagen of the upper layer is greater than 1.3 parts by weight with respect to 1 part by weight of the collagen of the lower structure, the concentration of the collagen of the upper layer is too higher than that of the collagen in the lower structure, thus exhibiting higher density, and thus in the processes of placing the upper layer and binding the upper layer to the lower structure, an interface between the upper layer and the lower structure becomes unclear due to the density difference, and the bone mineral included in the lower structure is introduced into the upper layer such that an extracellular matrix protein layer of the upper layer is unable to properly act as a barrier membrane, and thus this case is not suitable for use as an integrated biomaterial for bone tissue regeneration.

In the present invention, the upper layer including collagen preferably has a thickness of 20% to 35% of the entire biomaterial thickness. Preferably, the thickness of the upper layer is in the range of 0.5 mm to 1.5 mm and the thickness of the lower structure is in the range of 1 mm to 6 mm. More preferably, the thickness of the upper layer may be 1 mm and the thickness of the lower structure may range from 2 mm to 4 mm, but the present invention is not limited thereto.

The integrated biomaterial according to the present invention may further comprise an antimicrobial or anti-inflammatory functional material, and the antimicrobial or anti-inflammatory functional material may be, but is not limited to, any one or more selected from the group consisting of an antimicrobial agent, an antibiotic, and a peptide or protein with an anti-inflammatory function.

In the present invention, the antimicrobial agent may be, but is not limited to, sodium ethylenediaminetetraacetate, sodium copper chlorophyllin, a synthetic material containing fluorine or chlorine such as sodium fluoride or benzethonium chloride , , aromatic carboxylic acid including benzoic acid and the like, allantoin, or tocopherol acetate.

In the present invention, the antibiotic may be, but is not limited to, minocycline, tetracycline, doxycycline, chlorohexidine, ofloxacin, tinidazole, ketoconazole, or metronidazole.

The antimicrobial peptide may be a peptide derived from human β-defensin, and the antimicrobial peptide may be selected from peptides having the amino acid sequences of SEQ ID NOS: 1 to 3, but the present invention is not limited thereto.

SEQ ID NO: 1(BD3-3): G-K-C-S-T-R-G-R-K-C-C-R-R-K-K
SEQ ID NO: 2 (BD3-3-M1): G-K-C-S-T-R-G-R-K-C-M-R-R-K-K
SEQ ID NO: 3 (BD3-3-M2): G-K-C-S-T-R-G-R-K-M-C-R-R-K-K

**[Examples]**

Hereinafter, the present invention will be described in further detail with reference to the following examples. These examples are provided for illustrative purposes only, and it will be obvious to those of ordinary skill in the art that the scope of the present invention is not construed as being limited by these examples. Thus, the substantial scope of the present invention should be defined by the appended claims.

**Example 1: Integrated Biomaterial in which Lower Structure Consisting of Bovine Bone-derived Bone Mineral Particles and Collagen, and Collagen Upper Layer Are Integrated (integrated biomaterial: 7.7% collagen contained, collagen concentration of upper layer: 0.5%)**

Bovine bone-derived bone mineral particles were prepared to have a particle size of 0.4 mm to 0.8 mm. 27 g of a bone mineral component was mixed with 50 mL of 4.0% (w/v) pig skin-derived collagen (2 g collagen) solution dissolved in 0.5 M acetic acid, and a mold was filled with the resulting mixture, and the lower structure was aligned on a clean bench. Separately, 50 mL of a 0.5% (w/v) pig skin-derived collagen (0.25 g collagen) solution dissolved in 0.5 M acetic acid was dispensed on the mixture to form an upper collagen layer. The weight of the used collagen was 7.7% (w/w) of the total weight. After the upper collagen layer was dispensed, the layer was left on a clean bench for a certain period of time. The resulting layer was left in ammonia vapor saturated with 25% to 30% aqueous ammonia for 3 hours or longer to be gelled, and then washed with purified water to neutralize the pH.
After washing, the resulting product was frozen at - 1.5 °C for 2 hours or more, and then frozen to -20 °C at a freezing rate of 1 °C/min. After confirming that the integrated biomaterial was completely frozen at -20 °C, lyophilization was performed for 48 hours. The lyophilized integrated biomaterial was cross-linked in a vacuum oven at 140 °C for 120 hours, thereby completing the preparation of an integrated biomaterial.

**Example 2: Integrated Biomaterial in which Lower Structure Consisting of Bovine Bone-derived Bone Mineral Particles and Collagen, and Upper Collagen Layer Are Integrated (integrated biomaterial: 10.0% collagen contained, collagen concentration of upper layer: 2.0%)**

Bovine bone-derived bone mineral particles were prepared to have a particle size of 0.4 mm to 0.8 mm. 27 g of a bone mineral component was mixed with 50 mL of 4.0% (w/v) pig skin-derived collagen (2 g collagen) solution dissolved in 0.5 M acetic acid, and a mold was filled with the resulting mixture, and the lower structure was aligned on a clean bench. Separately, 50 mL of a 2.0% (w/v) pig skin-derived collagen (1 g collagen) solution dissolved in 0.5 M acetic acid was dispensed on the mixture to form an upper collagen layer. The weight of the used collagen was 10.0% (w/w) of the total weight. After the upper collagen layer was dispensed, the layer was left on a clean bench for a certain period of time. The resulting layer was left in ammonia vapor saturated with 25% to 30% aqueous ammonia for 3 hours or longer to be gelled, and then washed with purified water to neutralize the pH. After washing, the resulting product was frozen at -1.5 °C for 2 hours or more, and then frozen to -20 °C at a freezing rate of 1 °C/min. After confirming that the integrated biomaterial was completely frozen at -20 °C, lyophilization was performed for 48 hours. The lyophilized integrated biomaterial was cross-linked in a vacuum oven at 140 °C for 120 hours, thereby completing the preparation of an integrated biomaterial.

**Example 3: Integrated Biomaterial in which Lower Structure Consisting of Bovine Bone-derived Bone Mineral Particles and Collagen, and Upper Collagen Layer Are Integrated (integrated biomaterial: 14.3% collagen contained, collagen concentration of upper layer: 5.0%)**

Bovine bone-derived bone mineral particles were prepared to have a particle size of 0.4 mm to 0.8 mm. 27 g of a bone mineral component was mixed with 50 mL of 4.0% (w/v) pig skin-derived collagen (2 g collagen) solution dissolved in 0.5 M acetic acid, and a mold was filled with the resulting mixture, and the lower structure was aligned on a clean bench. Separately, 50 mL (2.5 g collagen) of a 5.0% (w/v) pig skin-derived collagen (2.5 g collagen) solution dissolved in 0.5 M acetic acid was dispensed on the mixture to form an upper collagen layer. The weight of the used collagen was 14.3% (w/w) of the total weight. After the upper collagen layer was dispensed, the layer was left on a clean bench for a certain period of time. The resulting layer was left in ammonia vapor saturated with 25% to 30% aqueous ammonia for 3 hours or longer to be gelled, and then washed with purified water to neutralize the pH. After washing, the resulting product was frozen at -1.5 °C for 2 hours or more, and then frozen to -20 °C at a freezing rate of 1 °C/min. After confirming that the integrated biomaterial was completely frozen at -20 °C, lyophilization was performed for 48 hours. The lyophilized integrated biomaterial was cross-linked in a vacuum oven at 140 °C for 120 hours, thereby completing the preparation of an integrated biomaterial.

**Comparative Example 1: Biomaterial Consisting of Lower Structure Only, which Consists of Bovine Bone-derived Bone Mineral Particles and Collagen (biomaterial: 6.90% collagen contained, collagen concentration of upper layer: 0%)**

Bovine bone-derived bone mineral particles were prepared to have a particle size of 0.4 mm to 0.8 mm. 27 g of a bone mineral component was mixed with 50 mL of 4.0% (w/v) pig skin-derived collagen (2 g collagen) solution dissolved in 0.5 M acetic acid, and a molding was filled with the resulting mixture, and the lower structure was aligned on a clean bench. The weight of the used collagen was 6.90% of the total weight. After the upper collagen layer was dispensed, the layer was left on a clean bench for a certain period of time. The upper collagen layer was gelled by a strong base, and then washed with purified water to neutralize the pH. After washing, the resulting product was frozen at -1.5 °C for 2 hours or more, and then frozen to -20 °C at a freezing rate of 1 °C/min. After confirming that the integrated biomaterial was completely frozen at - 20 °C, lyophilization was performed for 48 hours. The lyophilized integrated biomaterial was cross-linked in a vacuum oven at 140 °C for 120 hours, thereby completing preparation of an integrated biomaterial. The amounts of a bone graft material and collagen used are shown in Table 1 below.

[Table 1]

| Conditions Used amount | Example 1 | Example 2 | Example 3 | Comparative Example 1 |
|---|---|---|---|---|
| Amounts of bone graft material and collagen used in lower structure | | | | |
| Bone mineral (g) | 27 | 27 | 27 | 27 |
| Collagen (g) | 2 | 2 | 2 | 2 |
| Amount of collagen used in upper layer | | | | |
| Collagen (g) | 0.25 | 1 | 2.5 | 0 |
| Weight (%) of collagen with respect to total amount | 2.25/29.25*100 = 7.69 (%) | 3/30*100=10.0 (%) | 4.5/31.5*100=14.28 (%) | 2/29*100=6.90 (%) |
| (Amount of collagen used in upper and lower)/(amo unts of collagen in upper and lower and bone graft material) | (2+0.25)/ (2.25+27)* 100=7.69 (%) | (2+1)/(3+27) *10 0=10.0 (%) | (2+2.5)/(4.5+27)*10 0=14.28 (%) | (2-0)/(2+27)*10 0=6.90 (%) |

**Experimental Example 1: Observation of Structure of Prepared Integrated Biomaterial**

The integrated biomaterials prepared according to Examples 1 to 3 and our commercial product (OCS-B Xenomatrix Collagen, NIBEC, Korea) as a control were observed using a differential scanning electron microscope. Each tissue-structured mimetic was coated with platinum and observed with a field emission scanning electron microscope (FE-SEM, Jeol, S-4700, Japan).

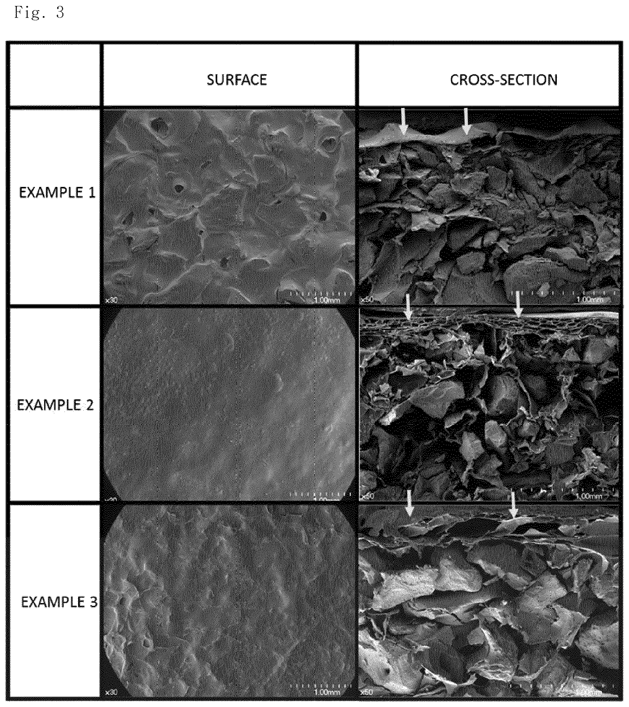

FIG. 3 is a set of differential scanning microscope images of the integrated biomaterials of Examples 1 to 3. It was observed that the collagen and bone mineral components were uniformly mixed in the composite structures prepared in Examples 1 to 3, and it was confirmed that the lower structure consisting of bone mineral and collagen and the upper collagen layer were firmly bound to each other without an empty space therebetween.

**Experimental Example 2: Test for Degradation by Collagenase**

20 ug/mL of collagenase (0.247 U/mg lyophilizate) was contained in an HBSS (Salt Solution) solution, and the integrated biomaterials of Examples 1 to 3 were left for a certain period of time and the degree of degradation thereof was examined.

Formula:

$$\text{Collagen degradation rate (\%)} = \frac{\text{difference } (a-b) \text{ between before and after degradability test}}{\text{Weight before degradability test}} \times 100$$

a: weight before degradability test (g)
b: weight after degradability test (g)
* Since the bone graft material is not degraded by collagenase, it does not affect collagen degradation rate.

After 2 weeks, collagen degradation activity in the upper collagen layer and the lower structure (a bone graft material and collagen mixed), which are the whole structure, was tested, and the results showed degradation of a maximum of at most 6.90% and at least 1% with respect to weight before the test. Considering that the degradation rate of collagen in the lower structure was 1% in the control, the degradation rate of collagen only in the upper layer of each of Examples 1 to 3 was 2.41% to 5.90% respectively obtained by subtraction of 1%.

It was confirmed that the upper collagen layer was retained until 2 weeks, and in Examples 2 and 3, the degradation rate of the upper collagen layer was maintained at 3% or less.

The degradation degree according to the concentration of collagenase is shown in Table 2 below.

[Table 2]

| Weight according to condition / Concentration of collagenase | Control | | | Collagen concentration of upper layer of integrated biomaterials according to examples | | | | | | | | |
|---|---|---|---|---|---|---|---|---|---|---|---|---|
| | 0% (Comparative Example 1) | | | 0.5% (Example 1) | | | 2% (Example 2) | | | 5% (Example 3) | | |
| | Initial weight (a) | Weight after 2 weeks (b) | Degradation rate *1 / Degradation rate *2 | Initial weight (a) | Weight after 2 weeks (b) | Degradation rate *1 | Initial weight (a) | Weight after 2 weeks (b) | Degradation rate *1 | Initial weight (a) | Weight after 2 weeks (b) | Degradation rate *1 |
| 20 µg/ml | 1.0190 (g) | 1.0088 (g) | 1.00% | 1.0507 (g) | 0.9782 (g) | 6.90% (*1) / 5.90% (*2) | 0.8508 (g) | 0.8218 (g) | 3.41% (*1) / 2.41% (*2) | 0.8710 (g) | 0.8400 (g) | 3.56% (*1) / 2.56% (*2) |

*1) Degradation rate of collagen in integrated structure (including both the upper collagen layer and the lower structure)

*2) Degradation rate of collagen used in preparation of upper collagen layer

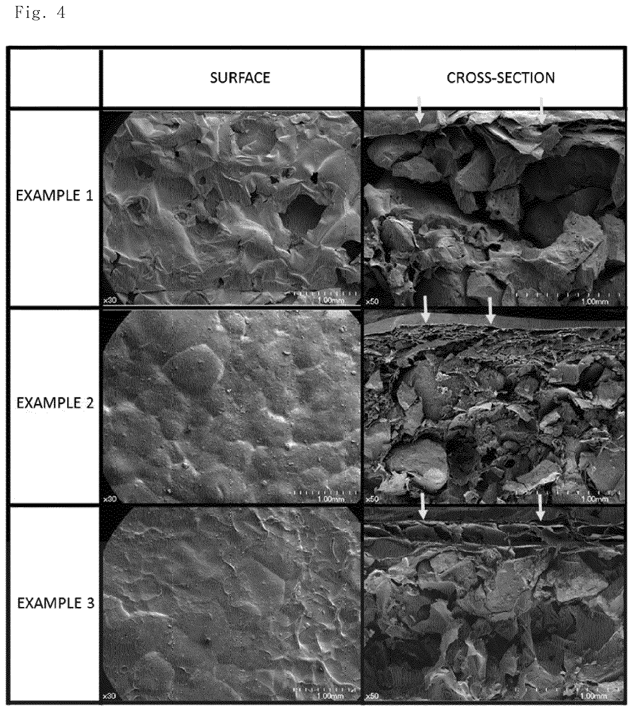

FIG. 4 illustrates differential scanning electron microscope images showing surfaces of the integrated biomaterials of Examples 1 to 3 and control, wherein the images were acquired after the degradation test was completed to identify the degree of degradation of collagen in the upper layer.

**Experimental Example 3: Test for Bone Regeneration in Animal**

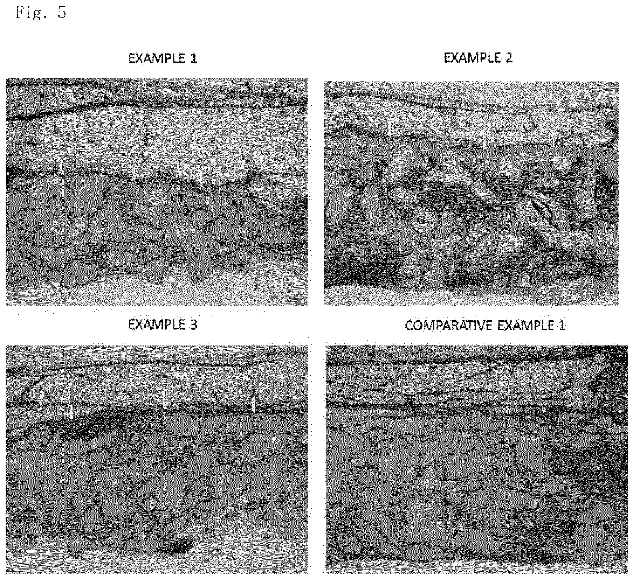

An 8 mm defect was formed in the skull of rabbits, and each of the integrated biomaterials of Examples 1 to 3 and the biomaterial of Comparative Example 1 as a control were implanted thereinto for 8 weeks, and then the degree of formation of new bone and the regeneration capacity of surrounding tissues were observed (see FIG. 5).

When the biomaterial of Example 2 was implanted, more new bone was formed than the other groups. In addition, the integrated biomaterial of Example 2 showed an increase in the area of a new bone by about 50% compared to Comparative Example 1. The histomorphometry results thereof are shown in Table 3 below.

[Table 3]

| Groups | New bone area (%) | Connective tissue area (%) | Bone graft area (%) |
|---|---|---|---|
| Comparative Example 1 | 10.81±8.49 | 62.35±11.26 | 26.84±8.92 |
| Example 1 | 12.85±7.63 | 65.14±12.35 | 22.01±7.21 |
| Example 2 | 20.13±6.63 | 56.95±7.96 | 22.92±9.49 |
| Example 3 | 16.58±10.52 | 58.54±11.32 | 24.88±9.79 |

While the present invention has been particularly described with reference to specific embodiments thereof, it will be obvious to those of ordinary skill in the art that these exemplary embodiments are provided for illustrative purposes only and are not intended to limit the scope of the present invention. Therefore, the substantial scope of the present invention should be defined by the appended claims and equivalents thereto.

[Industrial Applicability]

In an integrated biomaterial for bone tissue regeneration according to the present invention, a lower structure consisting of extracellular matrix protein and bone mineral components realizes a natural bone tissue environment, and thus facilitates the regeneration of a new bone, and particularly, an upper layer consisting of an extracellular matrix protein is placed at an appropriate ratio, and thus not only prevents the infiltration of epithelial tissue or connective tissue but also maximizes bone tissue regeneration capability.

**Claims**

1. An integrated biomaterial for bone tissue regeneration, the integrated biomaterial comprising: a lower structure comprising collagen and a bone mineral component; and an upper layer comprising collagen, wherein an amount ratio (weight ratio) of the collagen of the upper layer to the collagen of the lower structure ranges from 0.13-1.3: 1.

2. The integrated biomaterial according to claim 1, wherein the bone mineral component comprises one or more selected from the group consisting of a living organism-derived bone mineral powder originating from allogenic bone or xenogenic bone , synthetic hydroxyapatite, and tricalcium phosphate micropowder..

3. The integrated biomaterial according to claim 1, wherein a content of the bone mineral component ranges from 80 wt% to 95 wt% with respect to a total weight of the integrated biomaterial, or
   wherein a total content of the collagen of the lower structure and the collagen of the upper layer ranges from 5 wt% to 20 wt% with respect to a total weight of the integrated biomaterial.

4. The integrated biomaterial according to claim 1, further comprising an antimicrobial or anti-inflammatory functional material.

5. A method of preparing an integrated biomaterial for bone tissue regeneration according to claim 1, the method comprising the following processes:

   (a) molding a lower structure mixture comprising collagen and bone mineral particles;
   (b) aligning a structure of the lower structure mixture comprising collagen and bone mineral particles;
   (c) placing an upper layer comprising collagen thereon;
   (d) binding the upper layer and the lower structure;

(e) lyophilizing the resulting structure; and
(f) thermally cross-linking the collagen of the upper layer, wherein an amount ratio (weight ratio) of the collagen of the upper layer to the collagen of the lower structure ranges from 0.13-1.3: 1.

6. The method according to claim 5, wherein the bone mineral component comprises one or more selected from the group consisting of a living organism-derived bone mineral powder originating from allogenic bone or xenogenic bone, synthetic hydroxyapatite, and tricalcium phosphate micropowder.

7. The method according to claim 5, wherein a content of the bone mineral component ranges from 80 wt% to 95 wt% with respect to a total weight of the integrated biomaterial.

8. The method according to claim 5, wherein a total content of the collagen of the lower structure and the collagen of the upper layer ranges from 5 wt% to 20 wt% with respect to a total weight of the integrated biomaterial.

9. The method according to claim 5, wherein the upper layer and the lower structure of process (d) are bound through gelation using a strong base.

10. The method according to claim 5, wherein process (e) is performed by thermal crosslinking at 140°C to 160 °C for 48 hours to 168 hours.

11. The method according to claim 5, further comprising, after process (e):

(g) adding an antimicrobial or anti-inflammatory functional material; and
(h) lyophilizing the resulting structure.


**Patentansprüche**

1. Integriertes Biomaterial zur Regeneration von Knochengewebe, wobei das integrierte Biomaterial Folgendes umfasst: eine untere Struktur, die Kollagen und eine Knochenmineralkomponente umfasst; und eine obere Schicht, die Kollagen umfasst, wobei ein Mengenverhältnis (Gewichtsverhältnis) des Kollagens der oberen Schicht zu dem Kollagen der unteren Struktur im Bereich von 0,13-1,3:1 liegt.

2. Integriertes Biomaterial nach Anspruch 1, wobei die Knochenmineralkomponente eine oder mehrere umfasst, die aus der aus Folgendem bestehenden Gruppe ausgewählt ist/sind: einem aus einem Lebendorganismus abgeleiteten Knochenmineralpulver, das aus allogenem Knochen oder xenogenem Knochen stammt, synthetischem Hydroxyapatit und Tricalciumphosphatmikropulver.

3. Integriertes Biomaterial nach Anspruch 1, wobei der Gehalt der Knochenmineralkomponente im Bereich von 80 Gew.-% bis 95 Gew.-% bezogen auf das Gesamtgewicht des integrierten Biomaterials liegt oder wobei der Gesamtgehalt des Kollagens der unteren Struktur und des Kollagens der oberen Schicht im Bereich von 5 Gew.-% bis 20 Gew.-% bezogen auf das Gesamtgewicht des integrierten Biomaterials liegt.

4. Integriertes Biomaterial nach Anspruch 1, das außerdem ein antimikrobielles oder entzündungshemmendes funktionelles Material umfasst.

5. Verfahren zur Herstellung eines integrierten Biomaterials zur Regeneration von Knochengewebe nach Anspruch 1, wobei das Verfahren die folgenden Vorgänge umfasst:

(a) das Formen eines unteren Strukturgemisches, das Kollagen und Knochenmineralteilchen umfasst;
(b) das Ausrichten einer Struktur des unteren Strukturgemisches, das Kollagen und Knochenmineralteilchen umfasst;
(c) das Platzieren einer oberen Schicht darauf, die Kollagen umfasst;
(d) das Verbinden der oberen Schicht und der unteren Struktur,
(e) das Gefriertrocknen der resultierenden Struktur; und
(f) das thermische Vernetzen des Kollagens der oberen Schicht, wobei ein Mengenverhältnis (Gewichtsverhältnis) des Kollagens der oberen Schicht zu dem Kollagen der unteren Struktur im Bereich von 0,13-1,3:1 liegt.

**6.** Verfahren nach Anspruch 5, wobei die Knochenmineralteilchen eine oder mehrere umfasst, die aus der aus Folgendem bestehenden Gruppe ausgewählt ist/sind: einem aus einem Lebendorganismus abgeleiteten Knochenmineralpulver, das aus allogenem Knochen oder xenogenem Knochen stammt, synthetischem Hydroxyapatit und Tricalciumphosphatmikropulver.

**7.** Verfahren nach Anspruch 5, wobei der Gehalt der Knochenmineralkomponente im Bereich von 80 Gew.-% bis 95 Gew.-% bezogen auf das Gesamtgewicht des integrierten Biomaterials liegt.

**8.** Verfahren nach Anspruch 5, wobei der Gesamtgehalt des Kollagens der unteren Struktur und des Kollagens der oberen Schicht im Bereich von 5 Gew.-% bis 20 Gew.-% bezogen auf das Gesamtgewicht des integrierten Biomaterials liegt.

**9.** Verfahren nach Anspruch 5, wobei die obere Schicht und die untere Struktur von Vorgang (d) mittels Gelierung unter Verwendung einer starken Base verbunden werden.

**10.** Verfahren nach Anspruch 5, wobei Vorgang (e) durch thermisches Vernetzen bei 140 °C bis 160 °C über 48 h bis 168 h durchgeführt wird.

**11.** Verfahren nach Anspruch 5, das nach Vorgang (e) außerdem Folgendes umfasst:

(g) das Hinzufügen eines antimikrobiellen oder entzündungshemmenden funktionellen Materials; und
(h) das Gefriertrocknen der resultierenden Struktur.

## Revendications

**1.** Biomatériau intégré pour une régénération de tissu osseux, le biomatériau intégré comprenant : une structure inférieure comprenant du collagène et un composant minéral osseux ; et une couche supérieure comprenant du collagène, dans lequel un rapport de quantité (rapport de poids) du collagène de la couche supérieure au collagène de la structure inférieure est compris entre 0,13 et 1,3 : 1.

**2.** Biomatériau intégré selon la revendication 1, dans lequel le composant minéral osseux comprend un ou plusieurs éléments choisis dans le groupe constitué d'une poudre minérale osseuse dérivée d'un organisme vivant provenant d'un os allogénique ou d'un os xénogénique, d'hydroxyapatite synthétique et d'une micro-poudre de phosphate tricalcique.

**3.** Biomatériau intégré selon la revendication 1, dans lequel une teneur du composant minéral osseux est dans la plage de 80 % en poids à 95 % en poids par rapport au poids total du biomatériau intégré, ou
dans lequel une teneur totale du collagène de la structure inférieure et du collagène de la couche supérieure est comprise entre 5 % en poids et 20 % en poids par rapport au poids total du biomatériau intégré.

**4.** Biomatériau intégré selon la revendication 1, comprenant en outre un matériau fonctionnel antimicrobien ou anti-inflammatoire.

**5.** Procédé de préparation d'un biomatériau intégré pour une régénération de tissu osseux selon la revendication 1, le procédé comprenant les processus suivants :

(a) mouler un mélange de structure inférieure comprenant du collagène et des particules minérales osseuses ;
(b) aligner une structure du mélange de structure inférieure comprenant du collagène et des particules minérales osseuses ;
(c) placer une couche supérieure comprenant du collagène sur celle-ci ;
(d) lier la couche supérieure et la structure inférieure ;
(e) lyophiliser la structure résultante ; et
(f) réticuler thermiquement le collagène de la couche supérieure, dans lequel un rapport de quantité (rapport de poids) du collagène de la couche supérieure au collagène de la structure inférieure est dans la plage de 0,13 et 1,3 : 1.

**6.** Procédé selon la revendication 5, dans lequel le composant minéral osseux comprend un ou plusieurs choisis dans le groupe constitué d'une poudre minérale osseuse dérivée d'un organisme vivant provenant d'un os allogénique ou d'un os xénogénique,
d'hydroxyapatite synthétique et d'une micro-poudre de phosphate tricalcique.

7. Procédé selon la revendication 5, dans lequel une teneur du composant minéral osseux est dans la plage de 80 % en poids à 95 % en poids par rapport au poids total du biomatériau intégré.

8. Procédé selon la revendication 5, dans lequel une teneur totale du collagène de la structure inférieure et du collagène de la couche supérieure est dans la plage de 5 % en poids à 20 % en poids par rapport au poids total du biomatériau intégré.

9. Procédé selon la revendication 5, dans lequel la couche supérieure et la structure inférieure du processus (d) sont liées par gélification en utilisant une base forte.

10. Procédé selon la revendication 5, dans lequel le processus (e) est effectué par réticulation thermique entre 140°C et 160°C pendant 48 heures à 168 heures.

11. Procédé selon la revendication 5, comprenant en outre, après le processus (e), les étapes consistant à :

g) ajouter un matériau fonctionnel antimicrobien ou anti-inflammatoire ; et
(h) lyophiliser la structure résultante.

Fig. 1

Before treatment     Place Bone substitute     Place membrane     After suture

Fig. 2

(a)

Upper layer (barrier membrane function)

Lower structure (bone tissue-like structure)

(b)

Upper layer

Lower part (bone mineral and extracellular matrix)

x100     500um

(C)

**Separate membrane and bone graft**      **Assembled membrane and bone graft**

Connective tissue migration

New bone ingrowth

Defect site

Prevention of connective tissue migration

New bone ingrowth

Defect site

Fig. 3

| | SURFACE | CROSS-SECTION |
|---|---|---|
| EXAMPLE 1 | | |
| EXAMPLE 2 | | |
| EXAMPLE 3 | | |

Fig. 4

| | SURFACE | CROSS-SECTION |
|---|---|---|
| EXAMPLE 1 | | |
| EXAMPLE 2 | | |
| EXAMPLE 3 | | |

Fig. 5

EXAMPLE 1

EXAMPLE 2

EXAMPLE 3

COMPARATIVE EXAMPLE 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016106885 A1

**Non-patent literature cited in the description**

- **J. GOTTLOW et al.** *J. Clin. Perio,* 1986, vol. 13, 604-616

- **ANSELME.** Osteoblast adhesion on biomaterials. *Biomaterials,* 2000, vol. 21 (7), 667-81